# EUROPEAN PATENT APPLICATION

(11) **EP 4 527 452 A1**
(43) Date of publication of application: **26.03.2025**
(21) Application number: 23824228.3
(22) Date of filing: 14.06.2023
(51) Int. Cl.: A61N 7/02, A61N 7/00

(54) **ULTRASOUND GENERATION DEVICE**

(30) Priority: 14.06.2022 KR 20220072247; 12.06.2023 KR 20230074950
(71) Applicant: Jeisys Medical Inc., Geumcheon-gu Seoul 08501 (KR)
(72) Inventor: SUN, Hye Jin, Seoul 08501 (KR); KANG, Dong Hwan, Seoul 08501 (KR)
(74) Representative: dompatent von Kreisler Selting Werner - Partnerschaft von Patent- und Rechtsanwälten mbB
(86) International application number: PCT/KR2023/008199
(87) International publication number: WO 2023/244014

(57) **Abstract**

The present disclosure relates to an ultrasound generation device comprising: an ultrasound generation unit for irradiating the skin with ultrasound waves; and a control unit for controlling the operation of the ultrasound generation unit, wherein the control unit controls the ultrasound generation unit so that the ultrasound generation unit applies a specific ultrasound energy corresponding to at least one from among a fat increase, a fat reduction, and an elastic fiber increase in a skin layer.

## Description

### [TECHNICAL FIELD]

The present disclosure relates to an ultrasound generation device. More specifically, the present disclosure relates to an ultrasound generation device that can selectively provide an increase or decrease in a subcutaneous fat layer, or an enhancement of elasticity, depending on the applied ultrasound energy when ultrasound energy is applied.

### [BACKGROUND ART]

Ultrasound refers to waves with a frequency of 20 kHz or higher, and has the property of penetrating water, so it is widely used in the medical field, such as ultrasound diagnostic devices and ultrasound treatment devices.

The most representative application of ultrasound in the medical field is an ultrasound imaging device that utilizes the properties of ultrasound penetration and reflection. For example, there is a device that visualizes the time and intensity of reflection as ultrasound penetrates the human body and passes through each organ, thereby obtaining a cross-sectional image of the human body.

In addition, there is a device that uses the heat generated by high-intensity focused ultrasound (HIFU) to burn and remove specific subcutaneous tissues, such as tumors in the skin, or to induce degeneration and regeneration of skin tissue, resulting in skin beauty or skin plastic surgery effects, such as wrinkle improvement.

Currently, the ultrasound generation devices in the skin beauty market are used for lifting procedures by focusing ultrasound energy on the SMAS layer of the skin, or for fat reduction procedures by focusing strong energy on the subcutaneous fat layer to destroy fat cells.

These ultrasound generation devices can focus ultrasound at a desired depth and adjust the desired depth. They are used to deliver effective energy to a desired depth without damaging the epidermis.

There are two methods for delivering energy with the ultrasound generation device: the dot method and the linear method. The dot method is the most widely used method, and energy is applied at a stationary location while the transducer moves and stops repeatedly, and energy is applied in the form of one or more fixed points. The linear method is a method in which energy is continuously applied while the transducer moves a specific distance at a constant speed, thereby creating linear heat formation in the skin tissue.

The dot type is a classically used method and is widely used for lifting, and the linear type is effectively used for fat reduction because it allows bulk heating.

However, when applying strong ultrasonic energy in the dot type and linear type, epidermal burns can occur, and inflammatory substances can be generated, causing problems in the dermis layer, which are caused by excessive tissue coagulation.

In addition, when performing ultrasound treatment with strong energy like the conventional method in an area where there is no fat layer, the problem of further reduction in the fat layer often occurs, or ultrasound treatment is not recommended for people with thin fat layers.

Therefore, research is needed to find a range of energy and methods that not only reduce fat in a desired area with ultrasound, but also promote elasticity and increase the fat layer.

### [DETAILED DESCRIPTION OF THE INVENTION]

### [TECHINICAL PROBLEM]

The embodiment disclosed in the present disclosure is to improve elasticity of a dermal layer or selectively provide an increase or decrease in a subcutaneous fat layer depending on the specific ultrasonic energy applied when ultrasonic energy is applied.

Technical problems of the inventive concept are not limited to the technical problems mentioned above, and other technical problems not mentioned will be clearly understood by those skilled in the art from the following description.

### [TECHNICAL SOLUTION]

In an aspect of the present disclosure, an ultrasound generation device may include an ultrasound generator configured to irradiate ultrasound to a skin; and a controller configured to control an operation of the ultrasound generator, wherein the controller is configured to control the ultrasound generator to apply specific ultrasound energy corresponding to at least one of fat increase, fat reduction, and elastic fiber increase of a skin layer.

Furthermore, the ultrasound generator may be configured to generate a focus point at a vertical distance of 0.1 mm to 13 mm from a surface of the skin for at least one of the fat increase, the fat reduction, and the elastic fiber increase, and the focus point may include a dot type in which the focus point is generated according to a predetermined interval between the focus points while the focus point moves in a horizontal direction, and a linear type in which there is no distance between the focus points.

Furthermore, the controller may be configured to control the ultrasound generator to apply specific ultrasound energy of 0.1 J to 3 J in a frequency range of 2 MHz to 20 MHz.

Furthermore, the controller may be configured to control the ultrasound generator to apply ultrasound energy of 0.1 J to 0.3 J to a fat layer in the dot type of the ultrasound generator for the fat increase.

Furthermore, the controller may be configured to control the ultrasound generator to apply 0.2 J to 0.6 J of ultrasound energy to a fat layer or the fat layer from sub dermis in the dot type of the ultrasound generator for the fat reduction, or apply 0.2 J to 0.6 J of ultrasound energy to the fat layer or the sub dermis in the linear type for the fat reduction.

Furthermore, the controller may be configured to control the ultrasound generator to apply 0.2 J to 0.6 J of ultrasound energy to a dermis layer in the linear type of the ultrasound generator for the elastic fiber increase.

Furthermore, the controller may be configured to control the ultrasound generator to apply 0.1J to 0.3J of ultrasound energy to a fat layer in the dot type of the ultrasound generator, and apply 0.2J to 0.6J of ultrasound energy to a dermis layer in the linear type of the ultrasound generator for the fat increase and the elastic fiber increase.

Furthermore, the controller may be configured to control the ultrasound generator to apply 0.1 J to 0.6 J of ultrasound energy to a fat layer or the fat layer from sub dermis in the linear type of the ultrasound generator when the ultrasound generator moves to a specific point, and apply 0.2 J to 0.6 J of ultrasound energy to a dermis layer in the dot type of the ultrasound generator when the ultrasound generator moves back to an origin, or apply 0.1 J to 0.6 J of ultrasound energy to the fat layer or the fat layer from sub dermis in the dot type of the ultrasound generator when the ultrasound generator moves to a specific point, and apply 0.2 J to 0.6 J of ultrasound energy to a dermis layer in the linear type of the ultrasound generator when the ultrasound generator moves back to the origin, for the fat increase and the elastic fiber increase.

Furthermore, the ultrasound generator may be configured to irradiate the ultrasound to 0.1 mm to 13 mm of the skin layer and a fat layer with a frequency band of 2 MHz, 4 MHz, 5.5 MHz, 7 MHz, 10 MHz, and 20 MHz.

### [ADVANTAGEOUS EFFECTS OF THE INVENTION]

According to the above-described problem solving means of the present disclosure, when ultrasonic energy is applied, an effect is provided that can selectively improve the elasticity of the dermal layer or provide an increase or decrease in the subcutaneous fat layer depending on the specific ultrasonic energy applied.

The effects of the present disclosure are not limited to the effects mentioned above, and other effects that are not mentioned will be clearly understood by those skilled in the art from the description.

### [DESCRIPTION OF THE DRAWINGS]

FIG. 1 is a diagram illustrating a configuration of an ultrasound generation device according to the present disclosure.
FIG. 2 is a diagram illustrating an example of a process in which the ultrasound generator irradiates ultrasound.
FIG. 3 is a tissue photograph illustrating a result of fat reduction and fat increase in an obese rat model using the ultrasound generation device of FIG. 1.
FIG. 4 is a graph illustrating a result of an experiment using the ultrasound generation device of FIG. 1 in an obese rat model, with Depth1 being the 1 mm deep upper dermis, Depth2 being the 2 mm deep dermis layer, and Depth3 being the 3 mm deep fat layer.
FIG. 5 is a diagram illustrating a result of elasticity improvement and fat reduction and fat increase in an obese rat model using the ultrasound generation device of FIG. 1.
FIG. 6 is a graph illustrating a result of inflammatory substance expression in an obese rat model using the ultrasound generation device of FIG. 1.
FIG. 7 is a diagram illustrating a result of elasticity improvement in an obese rat model using the ultrasound generation device of FIG. 1.

### [BEST MODE]

In the drawings, the same reference numeral refers to the same element. This disclosure does not describe all elements of embodiments, and general contents in the technical field to which the present disclosure belongs or repeated contents of the embodiments will be omitted. The terms, such as "unit, module, member, and block" may be embodied as hardware or software, and a plurality of "units, modules, members, and blocks" may be implemented as one element, or a unit, a module, a member, or a block may include a plurality of elements.

Throughout this specification, when a part is referred to as being "connected" to another part, this includes "direct connection" and "indirect connection", and the indirect connection may include connection via a wireless communication network. Furthermore, when a certain part "includes" a certain element, other elements are not excluded unless explicitly described otherwise, and other elements may in fact be included.

Furthermore, when a certain part "includes" a certain element, other elements are not excluded unless explicitly described otherwise, and other elements may in fact be included.

In the entire specification of the present disclosure, when any member is located "on" another member, this includes a case in which still another member is present between both members as well as a case in which one member is in contact with another member.

The terms "first," "second," and the like are just to distinguish an element from any other element, and elements are not limited by the terms.

The singular form of the elements may be understood into the plural form unless otherwise specifically stated in the context.

Identification codes in each operation are used not for describing the order of the operations but for convenience of description, and the operations may be implemented differently from the order described unless there is a specific order explicitly described in the context.

Hereinafter, operation principles and embodiments of the present disclosure will be described with reference to the accompanying drawings.

First, High Intensity Focused Ultrasound (HIFU) technology is the latest thermal ablation treatment that burns specific subcutaneous tissues such as tumors in the skin by using the heat generated when high-intensity ultrasound is focused on one point in the skin. This is similar to the principle of focusing warm sunlight with a magnifying glass to light a fire. Since ultrasound easily passes through body tissues, HIFU treatment is performed in a completely non-invasive manner without a knife or even a needle. In other words, by simply pressing the patient's skin on the ultrasound generation surface, specific subcutaneous tissues such as tumors are burned and treated. In addition, HIFU treatment is currently being used to treat uterine fibroids, bone metastases, prostate cancer, breast cancer, pancreatic cancer, liver cancer, and kidney cancer.

This high-intensity focused ultrasound technology may be implemented through an ultrasound generation device. An ultrasound generation device can irradiate ultrasound to the surface of a patient's skin.

The controller of the ultrasound generation device according to the present disclosure in this specification includes various devices that may perform computational processing and provide results to a user. For example, the controller of the ultrasound generation device according to the present disclosure may include a computer, a server device, and a portable terminal, or may be in the form of one of them.

Here, the computer may include, for example, a notebook, a desktop, a laptop, a tablet PC, a slate PC, and the like mounted with a web browser.

The server device is a server that communicates with an external device to process information, and may include an application server, a computing server, a database server, a file server, a mail server, a proxy server, and a web server.

A portable terminal is a wireless communication device that ensures portability and mobility, and may include all kinds of handheld-based wireless communication devices such as PCS (Personal Communication System), GSM (Global System for Mobile communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (W-Code Division Multiple Access), WiBro (Wireless Broadband Internet) terminal, a smart phone, and the like, and a wearable device such as at least one of a watch, a ring, bracelets, anklets, a necklace, glasses, contact lenses, or a head-mounted device (HMD).

The ultrasound generation device according to the present disclosure may control an ultrasound generator to apply specific ultrasound energy corresponding to at least one of fat increase, fat decrease, and elastic fiber increase of the skin layer or fat layer.

The ultrasound generation device may selectively provide an effect of increasing or decreasing the subcutaneous fat layer or improving the elasticity of the skin layer according to the specific ultrasound energy applied when ultrasound energy is applied.

Hereinafter, the ultrasound generation device will be described in detail.

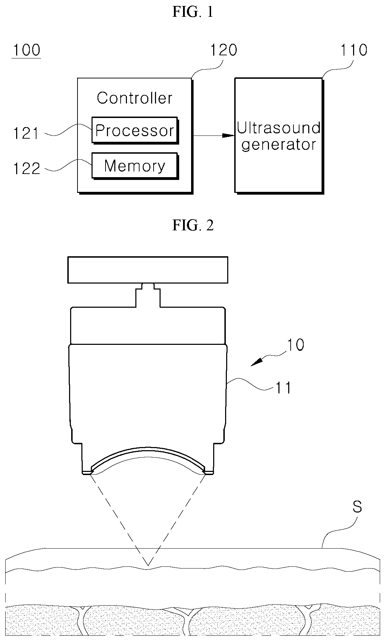

FIG. 1 is a diagram illustrating a configuration of an ultrasound generation device according to the present disclosure. FIG. 2 is a diagram illustrating an example of a process in which the ultrasound generator irradiates ultrasound.

Referring to FIGS. 1 and 2, an ultrasound generation device 100 may include an ultrasound generator 110 and a controller 120.

The ultrasound generator 110 having a transducer 11 may irradiate ultrasound to a skin S while maintaining the ultrasound focus depth.

The controller 120 may be implemented with a memory 122 that stores data on an algorithm for controlling the operation of components within the device or a program that reproduces the algorithm, and at least one processor 121 that performs the operation using the data stored in the memory 122. Here, the memory 122 and the processor 121 may be implemented as separate chips, respectively. In addition, the memory 122 and the processor 121 may be implemented as a single chip.

The memory 122 may store data supporting various functions of the device, programs for the operation of the control unit, may store input/output data, a plurality of application programs (or applications) executed on the device, data for the operation of the device, and commands. At least some of these application programs may be downloaded from an external server via wireless communication.

The memory 122 may include at least one type of storage medium among a flash memory type, a hard disk type, an SSD type (Solid State Disk type), an SDD type (Silicon Disk Drive type), a multimedia card micro type, a card type memory (for example, an SD or XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. In addition, the memory 122 may be a database that is separate from the device but connected by wire or wirelessly.

The processor 121 may control the ultrasound generator 110 so that the ultrasound generator 110 applies specific ultrasound energy corresponding to at least one of fat increase, fat reduction, or elastic fiber increase of the skin layer. At this time, the processor 121 may control the ultrasound generator 110 so that the ultrasound generator 110 applies specific ultrasound energy corresponding to at least one of improving elasticity of the skin layer or fat increase or fat reduction of the subcutaneous fat layer.

The ultrasound generator 110 may generate a focus point at a vertical distance of 0.1 mm to 13 mm from a skin surface for at least one of the fat increase, the fat reduction, or the elastic fiber (collagen, elastin fiber) increase. At this time, the ultrasound generator 110 may include a dot type in which a focus point is generated according to a predetermined interval between focus points while the focus point moves in a horizontal direction, and a linear type in which there is no distance between focus points.

The processor 121 may control the ultrasound generator 110 to apply specific ultrasound energy of 0.1 J to 3 J in a frequency range of 2 MHz to 20 MHz. For example, the ultrasound generator 110 may irradiate ultrasound to 0.1 mm to 13 mm of the skin layer and the fat layer in a frequency band of 2 MHz, 4 MHz, 5.5 MHz, 7 MHz, 10 MHz, and 20 MHz.

The processor 121 may control the ultrasound generator 110 to apply ultrasound energy of 0.1 J to 0.3 J to the fat layer in the dot type of the ultrasound generator 110 for the fat increase. For example, the doctor may control the ultrasound generator 110 to check a depth to be focused on the fat layer of the subject's examination area to a depth of 0.1 mm to 13 mm of the skin, and the processor 121 may control the ultrasound generator 110 to apply 0.2 J of ultrasound energy to the fat layer in the dot type of 2 MHz to 7 MHz.

The processor 121 may control the ultrasound generator 110 to apply 0.2 J to 0.6 J of ultrasound energy to the fat layer or the sub dermis in the dot type of the ultrasound generator 110 or to apply 0.2 J to 0.6 J of ultrasound energy to the fat layer or the sub-dermis in the linear type for fat reduction. For example, the doctor may check the depth to focus on the fat layer or the sub dermis of the subject's examination area to a depth of 0.1 mm to 13 mm of the skin, and the processor 121 may control the ultrasound generator 110 to apply 0.2 J to 0.6 J of ultrasound energy to the fat layer or the sub dermis of the subject's examination area in the dot type or the linear type of 2 MHz to 7 MHz.

The processor 121 may control the ultrasound generator 110 to apply 0.2 J to 0.6 J of ultrasound energy to the dermis lay in the linear type of the ultrasound generator 110 for the elastic fiber increase. For example, the doctor may control the ultrasound generator 110 to check the depth to be focused on the dermis layer of the subject's examination area to a depth of 0.1 mm to 13 mm of the skin, and the processor 121 may control the ultrasound generator 110 to apply 0.2 J to 0.6 J of ultrasound energy to the dermis layer in the linear type of 2 MHz to 20 MHz of the ultrasound generator 110.

The processor 121 may control the ultrasound generator 110 to apply 0.1 J to 0.3 J of ultrasound energy to the fat layer in the dot type of the ultrasound generator 110 and to apply 0.2 J to 0.6 J of ultrasound energy to the dermis layer in the linear type of the ultrasound generator 110 for the fat increase and the elastic fiber increase. For example, the doctor may control the ultrasound generator 110 to check the depth to be focused on the fat layer of the subject's examination area to a depth of 0.1 mm to 13 mm of the skin, and the processor 121 may control the ultrasound generator 110 to apply 0.1 J to 0.3 J of ultrasound energy to the fat layer in the dot type of 2 MHz to 20 MHz of the ultrasound generator 110. In addition, the doctor may control the ultrasound generator 110 to check the depth to be focused on the dermis layer of the subject's examination area to a depth of 0.1 mm to 13 mm of the skin, and the processor 121 may control the ultrasound generator 110 to apply 0.2 J to 0.6 J of ultrasound energy to the dermis layer in the linear type of 2 MHz to 20 MHz of the ultrasound generator 110.

The processor 121 may control the ultrasound generator 110 to apply 0.1 J to 0.6 J of ultrasound energy to the fat layer or the sub dermis in the linear type of the ultrasound generator 110 when the ultrasound generator 110 moves to a specific point for the fat reduction and the elastic fiber increase, and to apply 0.2 J to 0.6 J of ultrasound energy to the dermis layer in the dot type of the ultrasound generator 110 when the ultrasound generator 110 moves back to the origin. For example, the doctor may control the ultrasound generator 110 to check the depth to be focused on the fat layer or the sub dermis of the subject's examination area to a depth of 0.1 mm to 13 mm of the skin, and the processor 121 to apply 0.1 J to 0.6 J of ultrasound energy to the fat layer or the sub dermis of the subject's examination area in the linear type of 2 MHz to 20 MHz of the ultrasound generator 110 when the ultrasound generator 110 moves to a specific point, and to apply 0.2 J to 0.6 J of ultrasound energy to the dermis layer in the dot type of 2 MHz to 20 MHz of the ultrasound generator 110 when the ultrasound generator 110 moves back to the origin.

On the other hand, the processor 121 may control the ultrasound generator 110 to apply 0.1 J to 0.6 J of ultrasound energy to the fat layer or the sub dermis in the dot type of the ultrasound generator 110 when the ultrasound generator 110 moves to a specific point for the fat reduction and the elastic fiber increase, and to apply 0.2 J to 0.6 J of ultrasound energy to the dermis layer in the linear type of the ultrasound generator 110 when the ultrasound generator 110 moves back to the origin. For example, the doctor may control the ultrasound generator 110 to check the depth to be focused on the fat layer or the sub dermis of the subject's examination area at a depth of 0.1 mm to 13 mm of the skin, and the processor 121 to apply 0.1 J to 0.6 J of ultrasound energy to the fat layer or the sub dermis of the subject's examination area in the dot type of 2 MHz to 20 MHz of the ultrasound generator 110 when the ultrasound generator 110 moves to a specific point, and to apply 0.2 J to 0.6 J of ultrasound energy to the dermis layer in the linear type of 2 MHz to 20 MHz of the ultrasound generator 110 when the ultrasound generator 110 moves back to the origin.

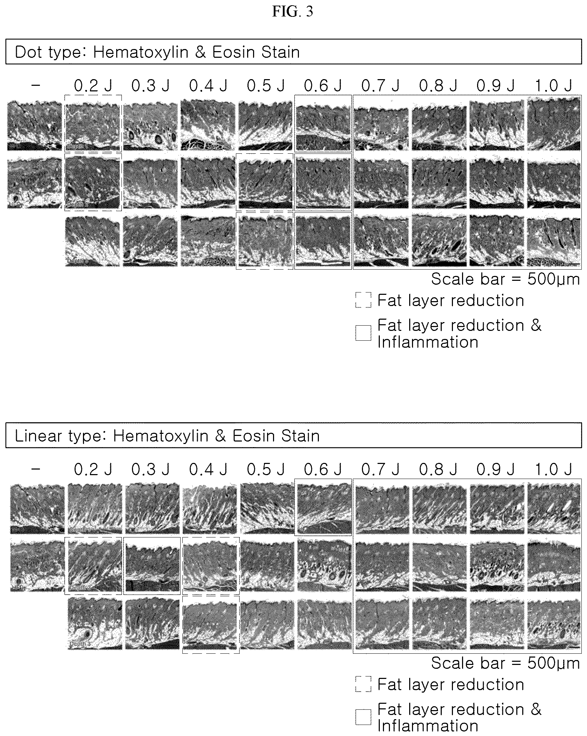

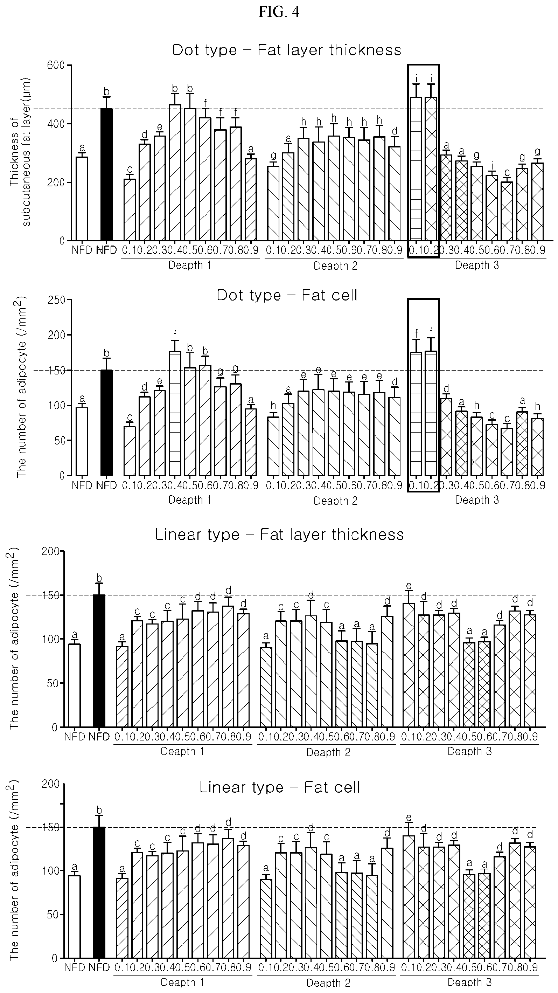

FIG. 3 is a tissue photograph illustrating a result of fat reduction and fat increase in an obese rat model using the ultrasound generation device of FIG. 1. FIG. 4 is a graph illustrating a result of an experiment using the ultrasound generation device of FIG. 1 in an obese rat model, with Depth1 being the 1 mm deep upper dermis, Depth2 being the 2 mm deep dermis layer, and Depth3 being the 3 mm deep fat layer.

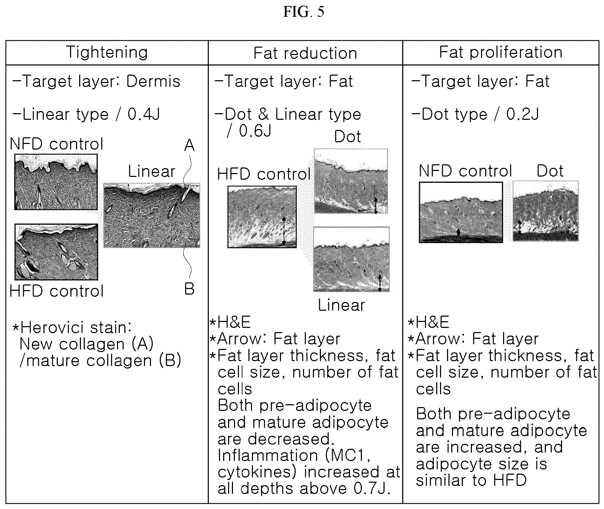

FIG. 5 is a diagram illustrating a result of elasticity improvement and fat reduction and fat increase in an obese rat model using the ultrasound generation device of FIG. 1.

When the dot type and the linear type of the ultrasound generator of FIG. 3 are applied at the same depth and with the same ultrasound energy, the tissue photograph illustrating the change in fat tissue are observed, and FIG. 4 quantifies the tissue result of FIG. 3 and shows the result as a graph. As a result of the tissue experiment in the obese rat model, when applying 0.2 J of ultrasound energy in the dot type to the fat layer and comparing it with the other conditions of FIG. 3, it is identified that it is effective in the fat increase. And, in the fat layer or the sub dermis, both the dot type and the linear type of the ultrasound generator of FIG. 3 applied 0.2J to 1.0J of ultrasound energy to the fat layer, and the fat reduction occurred, but from 0.7J, inflammatory substances are expressed, and it is identified that the most effective range is 0.2J to 0.6J by excluding these.

As shown in FIG. 5, in the tissue photographs, it is identified that the fat layer increased or decreased when ultrasound energy is applied with the dot type of the ultrasound generator of FIG. 3 and the linear type of the ultrasound generator of FIG. 4.

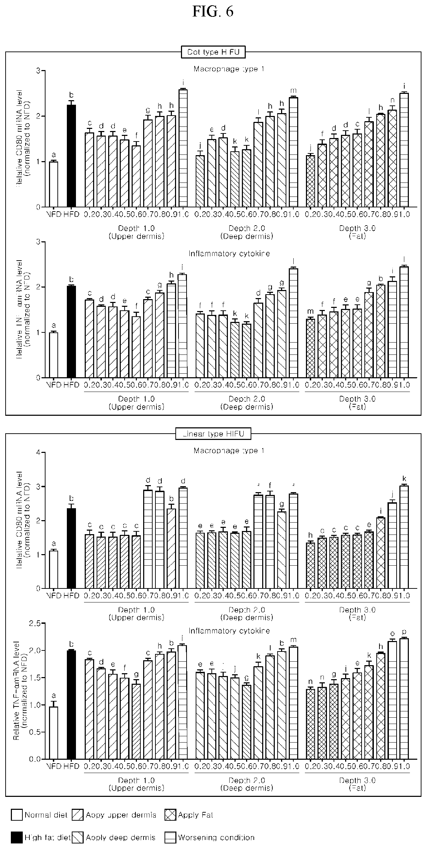

FIG. 6 is a graph illustrating a result of inflammatory substance expression in an obese rat model using the ultrasound generation device of FIG. 1.

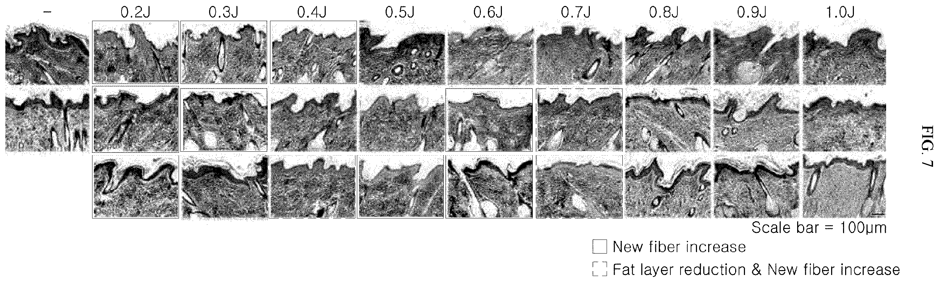

FIG. 6 is a graph of inflammation (Macrophage 1, inflammatory cytokine) level, and it is identified that the inflammation levels decreased from 0.2J to 0.6J in both the dot type of the ultrasound generator of FIG. 6 and the linear type of the ultrasound generator of FIG. 7, and increased from 0.7J or higher.

FIG. 7 is a diagram illustrating a result of elasticity improvement in an obese rat model using the ultrasound generation device of FIG. 1.

FIG. 7 is Herovici stain, and it is identified that the increase in new collagen and elastin fibers is identified, and it is identified that the increase is the greatest at 0.2J to 0.6J in the linear type.

Therefore, the ultrasound generation device according to the present disclosure may selectively provide an effect of increasing or decreasing a subcutaneous fat layer or improving the elasticity of a skin layer depending on the specific ultrasound energy applied and the skin layer applied when ultrasound energy is applied.

Meanwhile, the depth of the dermis layer and the fat layer are different depending on the person or the part, and the skin structure may be identified by measuring the relevant part with a skin measuring device, and a cartridge select one from 0.1 mm to 13 mm of a depth appropriate therefor may be selected.

At least one component may be added or deleted in accordance with the performance of the components illustrated in FIG. 1. In addition, it will be easily understood by those skilled in the art that the mutual positions of the components may be changed in accordance with the performance or structure of the system.

As described above, the disclosed embodiments have been described with reference to the attached drawings. A person skilled in the art will appreciate that the present disclosure may be practiced in other forms than the disclosed embodiments without changing the technical spirit or essential features of the present disclosure. The disclosed embodiments are illustrative and should not be construed as limiting.

## Claims

1. An ultrasound generation device, comprising:
an ultrasound generator configured to irradiate ultrasound to a skin; and
a controller configured to control an operation of the ultrasound generator,
wherein the controller is configured to control the ultrasound generator to apply specific ultrasound energy corresponding to at least one of fat increase, fat reduction, and elastic fiber increase of a skin layer.

2. The device according to claim 1,
wherein the ultrasound generator is configured to generate a focus point at a vertical distance of 0.1 mm to 13 mm from a surface of the skin for at least one of the fat increase, the fat reduction, and the elastic fiber increase, and
wherein the focus point includes a dot type in which the focus point is generated according to a predetermined interval between the focus points while the focus point moves in a horizontal direction, and a linear type in which there is no distance between the focus points.

3. The device according to claim 2,
wherein the controller is configured to control the ultrasound generator to apply specific ultrasound energy of 0.1 J to 3 J in a frequency range of 2 MHz to 20 MHz.

4. The device according to claim 3,
wherein the controller is configured to control the ultrasound generator to apply ultrasound energy of 0.1 J to 0.3 J to a fat layer in the dot type of the ultrasound generator for the fat increase.

5. The device according to claim 3,
wherein the controller is configured to control the ultrasound generator to apply 0.2 J to 0.6 J of ultrasound energy to a fat layer or the fat layer from sub dermis in the dot type of the ultrasound generator for the fat reduction, or apply 0.2 J to 0.6 J of ultrasound energy to the fat layer or the sub dermis in the linear type for the fat reduction.

6. The device according to claim 3,
wherein the controller is configured to control the ultrasound generator to apply 0.2 J to 0.6 J of ultrasound energy to a dermis layer in the linear type of the ultrasound generator for the elastic fiber increase.

7. The device according to claim 3,
wherein the controller is configured to control the ultrasound generator to apply 0.1J to 0.3J of ultrasound energy to a fat layer in the dot type of the ultrasound generator, and apply 0.2J to 0.6J of ultrasound energy to a dermis layer in the linear type of the ultrasound generator for the fat increase and the elastic fiber increase.

8. The device according to claim 3,
wherein the controller is configured to control the ultrasound generator to apply 0.1 J to 0.6 J of ultrasound energy to a fat layer or the fat layer from sub dermis in the linear type of the ultrasound generator when the ultrasound generator moves to a specific point, and apply 0.2 J to 0.6 J of ultrasound energy to a dermis layer in the dot type of the ultrasound generator when the ultrasound generator moves back to an origin, or
apply 0.1 J to 0.6 J of ultrasound energy to the fat layer or the fat layer from sub dermis in the dot type of the ultrasound generator when the ultrasound generator moves to a specific point, and apply 0.2 J to 0.6 J of ultrasound energy to a dermis layer in the linear type of the ultrasound generator when the ultrasound generator moves back to the origin, for the fat increase and the elastic fiber increase.

9. The device according to claim 2,
wherein the ultrasound generator is configured to irradiate the ultrasound to 0.1 mm to 13 mm of the skin layer and a fat layer with a frequency band of 2 MHz, 4 MHz, 5.5 MHz, 7 MHz, 10 MHz, and 20 MHz.
